# EUROPEAN PATENT APPLICATION

(11) **EP 4 552 659 A2**
(43) Date of publication of application: **14.05.2025**
(21) Application number: 25165613.8
(22) Date of filing: 03.04.2020
(51) Int. Cl.: A61L 2/26

(54) **METHOD TO TREAT LOOSE OBJECTS**

(30) Priority: 05.04.2019 IT 201900005154
(62) Divisional of application: 20167886.9
(71) Applicant: Icos Pharma S.p.A., 33080 Zoppola (IT)
(72) Inventor: Beni, Stefano, 33170 Pordenone (IT)
(74) Representative: Petraz, Gilberto Luigi

(57) **Abstract**

A method to treat loose objects comprises feeding untreated loose objects from a feed section (11) to a treatment machine (12); treating the untreated loose objects inside the treatment machine (12); unloading the treated loose objects from the treatment machine (12) into an unloading section (13) associated with a machine (14) that uses the treated loose objects.

## Description

### FIELD OF THE INVENTION

Embodiments described here concern a method to treat loose objects.

By treatment here we generally mean at least one cleaning, washing and/or sterilizing operation, with possible final drying and possible additional or intermediate treatments, or a combination of these or other suitable operations.

The loose products as above can be, for example, caps for closing test tubes for laboratory analysis, caps for closing drug vials, or small instruments or objects used in operating rooms or laboratories, or other.

### BACKGROUND OF THE INVENTION

It is known that in the hospital or pharmaceutical sector, or other areas, such as the cosmetic or food sector, it is necessary to use products that are suitably treated and controlled, for example sterilized, to prevent contaminations or alterations by substances with which they come into contact, or to prevent the introduction of bacteria, germs, other corpuscles or potentially pathogenic or harmful agents into environments where the total absence thereof is required.

Among the sterile products as above, in all the areas listed above, loose products, even of small sizes, are normally provided, such as, for example, caps for closing test tubes or vials intended to contain drugs, or substances to be subjected to laboratory analysis, or even small instruments or objects used in operating rooms or laboratories.

These loose products therefore need, in some cases, to be treated, and in particular sterilized. Some examples of machines or devices for sterilizing objects are described for example in documents DE-A-3841930, WO-A-2015150581 and EP-A-3064226.

In other documents, for example documents US-A-5397535, EP-A-0588145 and WO-A-2007014312, various operations for sterilizing or disinfecting objects are described, also used in the sanitary field or suchlike.

As is known, the loading of the loose products to be treated in the treatment chamber takes place by means of a feed section which cooperates with a loading aperture of the treatment chamber as above, to transfer into the latter a desired quantity of loose objects to be treated.

The loose products, at the end of the treatment cycle, are removed from an unloading mouth of the treatment chamber and transferred toward an unloading section, with which a user machine is generally operatively associated, for example a filling machine used in the pharmaceutical sector.

However, existing methods do not always guarantee the complete separation between untreated, and therefore contaminated, environments, such as for example the feed section, alternatively untreated and treated environments, that is, for example, on each occasion sterile and non-sterile, such as for example the treatment machine before and after the treatment has been carried out, and environments that have to always be kept treated, in particular sterile, such as for example the unloading zone operatively associated with the user machine, or the user machine itself.

In addition, over the years the need has arisen to obtain increasingly optimized, safe and controllable treatment methods, in particular in the field of application of the pharmaceutical industry.

In particular, the need has arisen to completely automate the steps of loading and unloading the loose objects into/from the treatment chamber and the step of transferring the loose objects from the treatment chamber to the filling machine in order to limit to a minimum, or even eliminate, human intervention in the methods to treat loose objects so as to substantially eliminate any possible source of external contamination.

There is therefore the need to perfect a method to treat loose objects that can overcome and improve the existing treatment methods.

In particular, one purpose of the present invention is to provide an optimized and efficient method to treat loose objects, which guarantees processing the loose objects, from when they are fed to when they are unloaded to the user machine, once treated, maintaining the desired controlled conditions of the environments in which the loose objects are processed.

Another purpose of the present invention is to provide a method to treat loose objects which guarantees to keep at least the environment of the unloading section operatively associated with the user machine controlled, both when it receives the loose objects at the end of the treatment cycle, and also when it supplies them to the user machine.

Another purpose of the present invention is to provide a method to treat loose objects which allows to increase the productivity and the quality of the treatment of the loose objects.

Yet another purpose of the present invention is to provide a method to treat loose objects which makes the operations of loading the loose objects into the treatment chamber and of unloading the loose objects, once treated, from the treatment chamber to the user machine quick and precise.

The Applicant has devised, tested and embodied the present invention to overcome the shortcomings of the state of the art and to obtain these and other purposes and advantages.

### SUMMARY OF THE INVENTION

The present invention is set forth and characterized in the independent claim. The dependent claims describe other characteristics of the invention or variants to the main inventive idea.

In accordance with the above purposes, a method to treat loose objects comprises feeding untreated loose objects from a feed section to a treatment machine, treating the untreated loose objects inside the treatment machine and unloading the treated loose objects from the treatment machine into an unloading section associated with a machine that uses the treated loose objects.

In accordance with one aspect, the method provides that before the treatment, when the treatment machine is in a condition in which it contains untreated loose objects, the treatment machine is put in communication, for the purpose of feeding, with the feed section and not with the unloading section.

Furthermore, the method provides that at the end of the treatment, when the treatment machine is in a condition in which it contains treated loose objects, the treatment machine is put in communication, for the purpose of unloading, with the unloading section and not with the feed section.

Advantageously, in this way it is guaranteed that the respective environments of the unloading section and of the treatment machine are in communication only when they are both in a treated condition, and that the respective environments of the treatment machine and of the feed section are in communication only when they are both in an untreated condition.

This solution is particularly useful in the industrial pharmaceutical sector in order to prevent contamination between sterile and non-sterile environments, guaranteeing, at the end of the treatment cycle, that the loose objects are correctly treated, for example sterilized, without the need for further interventions or processes.

Consequently, this method allows to improve the quality of the treatment of loose objects preventing any contamination between treated and untreated environments.

### ILLUSTRATION OF THE DRAWINGS

These and other characteristics of the present invention will become apparent from the following description of some embodiments, given as a non-restrictive example with reference to the attached drawings wherein:
- figs. 1-7 are example schematic illustrations of variants of an apparatus to treat loose objects to which the method to treat loose objects in accordance with the present invention is applied;
- fig. 8 is an example diagram of another variant of figs. 1-7;
- fig. 9 is an example diagram of another variant of figs. 1-7;
- fig. 9a is a variant of embodiments described here;
- fig. 10 is an example block diagram of a possible embodiment of the method to treat loose objects in accordance with the embodiments described here.

To facilitate comprehension, the same reference numbers have been used, where possible, to identify identical common elements in the drawings. It is understood that elements and characteristics of one embodiment can conveniently be incorporated into other embodiments without further clarifications.

### DESCRIPTION OF SOME EMBODIMENTS

We will now refer in detail to the various embodiments of the invention, of which one or more examples are shown in the attached drawings. Each example is supplied by way of illustration of the invention and shall not be understood as a limitation thereof. For example, the characteristics shown or described insomuch as they are part of one embodiment can be adopted on, or in association with, other embodiments to produce another embodiment. It is understood that the present invention shall include all such modifications and variants.

Before describing these embodiments, we must also clarify that the present description is not limited in its application to details of the construction and disposition of the components as described in the following description using the attached drawings. The present description can provide other embodiments and can be obtained or executed in various other ways. We must also clarify that the phraseology and terminology used here is for the purposes of description only, and cannot be considered as limitative.

One aspect of the present invention is a method to treat loose objects, described by way of example using the attached drawings 1-10.

With the terms "treatment", "treat" and "treated" we mean one or more washing and/or sterilizing operations, with possible final drying. In the context of washing, it is possible, for example, to carry out a pre-washing, washing with water or washing with chemical agents, combination of these washings, rinsing with hot or cold water. In the context of the treatment, a possible operation of thermal disinfection is also contemplated. In the context of the treatment, it is also possible to superficially treat the loose objects, for example by means of silicone coating; the silicone coating can be carried out for example in the context of the washing operation. Another possible example is a treatment that includes drying, typically by means of controlled air, for example sterile. For example, a treatment that includes washing and drying is possible. Yet another possible example of treatment provides washing, sterilization and drying. In these examples, the surface treatment of the loose objects, for example silicone coating, can be carried out at the same time or after washing, before the sterilization when provided. Yet another example of treatment provides sterilization and drying. Other combinations of the treatments as above can be contemplated in the embodiments described here.

The method described here can be applied to, or used on, an apparatus 10 to treat loose objects which comprises a section 11 for feeding loose objects to be treated, a treatment machine 12 and a section 13 for unloading the treated loose objects.

The feed section 11, the treatment machine 12 and the unloading section 13 can be disposed one after the other in sequence. In particular, the flow of loose objects to be treated is unidirectional from the feed section 11 to the treatment machine 12 and from the latter to the unloading section 13.

In some embodiments, the method according to the present description comprises:
- feeding untreated loose objects from the feed section 11 to the treatment machine 12;
- treating untreated loose objects inside the treatment machine 12;
- unloading the treated loose objects from the treatment machine 12 into an unloading section 13 associated with a machine 14 that uses the treated loose objects.

The method also provides that:
- before the treatment, when the treatment machine 12 is in a condition in which it contains untreated loose objects, the treatment machine 12 is put in communication, for the purpose of feeding, with the feed section 11 and not with the unloading section 13; and that
- at the end of the treatment, when the treatment machine 12 is in a condition in which it contains treated loose objects, the treatment machine 12 is put in communication, for the purpose of unloading, with the unloading section 13 and not with the feed section 11.

Advantageously, the method described here allows to keep separate environments which are normally treated and controlled, for example aseptic or sterile, such as the unloading section 13 associated with the user machine 14, from environments which are treated and untreated on each occasion, such as the treatment machine 12 respectively before and after the treatment of the loose objects has been carried out, and also from environments which are not normally treated, such as the feed section 11.

According to some embodiments, the steps of the method described here, and the sub-steps provided according to the embodiments described hereafter, can be executed, managed and controlled automatically by means of a suitable command and control unit 30. In this way, the method described here is automated and controlled, requiring minimal, if not zero, manual intervention. In particular, the command and control unit 30 on the basis of the state of operation of the user machine 14 can adjust the feeding, treatment and unloading steps, as described in detail below.

According to some embodiments, during the treatment of the loose objects the treatment machine 12 is separated, and therefore is not put in communication, at least with respect to the feed section 11 and possibly also with respect to the unloading section 13.

For example, in the event the treatment contemplates or includes sterilization, the feed section 11 is typically a non-sterile environment in which loose objects to be sterilized are present and along which they are transferred, while the unloading section 13 is a sterile environment, compatible with the user machine 14 with which it is combined and which, for intrinsic requirements and operational needs, is typically a treated and controlled environment, for example sterile, and where loose objects sterilized by the treatment machine 12 are present and along which they are transferred. Therefore, advantageously, in accordance with the present description, during the treatment method as above the unloading section 13 is never in communication, direct or indirect, with the feed section 11, in order to preserve the sterility of the unloading section 13 itself and of the user machine 14 connected downstream.

In particular, in possible embodiments, this selective separation and communication between the feed section 11, the treatment machine 12 and the unloading section 13, coordinated with the operating steps of the treatment described above, can be implemented by means of a suitable and synchronized selective opening/closing of closing and/or interception devices associated with the feed section 11, the treatment machine 12 and the unloading section 13 and/or with means or components that connect the feed section 11, the treatment machine 12 and the unloading section 13.

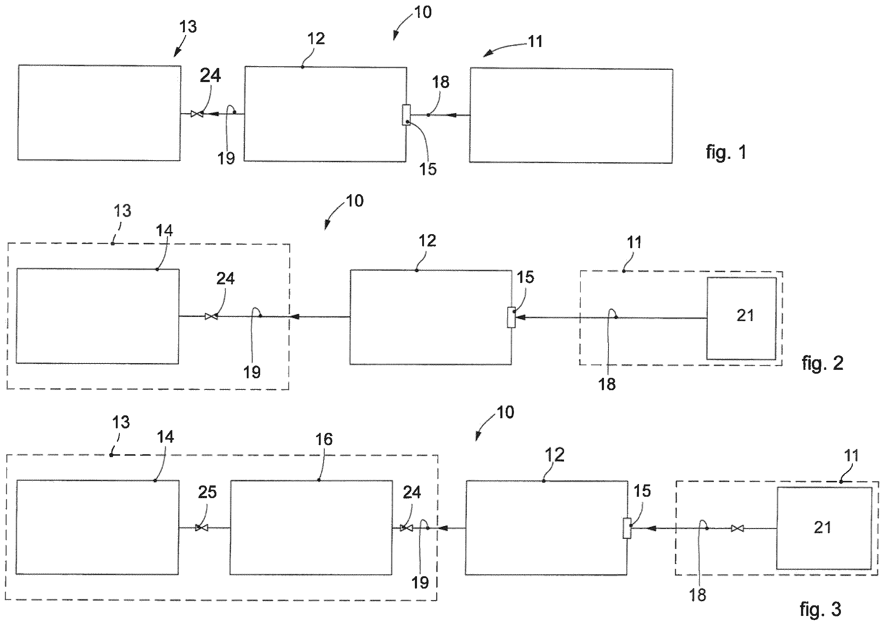

In particular, in some embodiments, the feed section 11 can be connected to the treatment machine 12 by means of feed means 18 (figs. 1-9), such as for example a feed conduit, by means of which the feeding step is carried out. The feed means 18 can be comprised in the feed section 11.

According to the method described here, it is provided to use a closing device 15 (fig. 1) associated with the treatment machine 12, which selectively closes the treatment machine 12 with a hermetic seal with respect to the feed section 11. This closed condition is maintained both during the treatment of the loose objects and also during the unloading thereof, once treated, toward the unloading section 13. The closing device 15, on the other hand, is opened when it is necessary to feed the loose objects to be treated from the feed section 11 to the treatment machine 12.

In accordance with some embodiments, as explained in detail below, the method can contemplate that the loose objects, before being transferred to the treatment machine 12, are suitably metered on each occasion into defined quantities and that these metered quantities are then transferred in succession to the treatment machine 12.

In accordance with other embodiments, the unloading section 13 is also connected to the treatment machine 12 by means of unloading means 19 (figs. 1-9), such as for example a respective unloading conduit, by means of which the unloading step is carried out. The unloading means 19 can be comprised in the unloading section 13.

The unloading section 13 can be associated, connected or integrated with a user machine 14 as described above.

In some embodiments, the user machine 14 can be a user machine normally configured to work continuously to process the treated loose objects that it receives, apart from operations to be performed during machine downtimes, for example maintenance, replacement of components or other, in coordination with the productivity requirements typical of the technical sector which is being operated in; for example, in the industrial pharmaceutical sector, but not only, there is a need for the user machine 14 to operate as continuously as possible, therefore providing that the feed requirements of the user machine 14 are always met.

Advantageously, in some embodiments, the method can provide to accumulate, in the unloading section 13, the treated loose objects unloaded from the treatment machine 12, before they are transferred to the user machine 14, so as to create a feed chamber and guarantee a continuous feed to the user machine 14. For this purpose, the unloading section 13 can comprise at least one intermediate accumulation container 16 (figs. 3-9) in which to accumulate the treated loose objects, before they are transferred to the user machine 14.

Possible embodiments of accumulation containers 16 are described by way of example in the international application WO 2018/225106 A2 in the name of the Applicant.

In accordance with other embodiments, as explained in detail below, the method can also contemplate that the treated loose objects, unloaded from the treatment machine 12, and for example accumulated in the accumulation container 16, are suitably metered on each occasion into defined quantities and that these metered quantities are then transferred in succession to the user machine 14, in accordance with the work cycle of the latter.

In other embodiments, the method can provide to unload the treated loose objects from the treatment machine 12 to the unloading section 13 and from there to the user machine 14, without accumulation; in this case, as described with reference, for example, to fig. 2, the accumulation container 16 may not be provided.

According to other embodiments, the method provides to carry out the selective unloading of the treated loose objects from the treatment machine 12 to the unloading section 13, selectively keeping the unloading section 13 separate from the treatment machine 12 when the latter is in the condition in which it contains untreated loose objects. To this end, the treatment apparatus 10, and in particular the unloading section 13, comprises an interception device 24 (figs. 1-9) located downstream of the treatment machine 12 and upstream of the accumulation container 16. The interception device 24 can be put in cooperation with the unloading means 19. The interception device 24 is provided to selectively enable the transit of the loose objects, treated in the treatment machine 12, from the treatment machine 12 to the user machine 14 and is suitable to selectively prevent the communication between the feed section 11 and the treatment machine 12 when the requirements of the method described here require it.

In these embodiments, during the unloading, the unloading section 13 is selectively put in communication with the treatment machine 12 by selective opening of the device 24 for intercepting the treated loose objects which is associated with the unloading means 19 that connect the treatment machine 12 with the unloading section 13.

In these embodiments, the method provides that the interception device 24 is selectively put in a closed condition in which it closes the unloading means 19, preventing the communication between the unloading section 13 and the treatment machine 12, and in an open condition in which it allows the communication between the unloading section 13 and the treatment machine 12 and in which the unloading of the treated loose objects can occur through the unloading means 19 from the treatment machine 12 to the unloading section 13.

In particular, during the unloading, the unloading section 13 is selectively put in communication with the treatment machine 12 by opening the interception device 24 when the closing device 15 is in the closed condition.

In possible applications of the present invention, the user machine 14 is a user machine 14 kept in controlled operating conditions, for example sterile, that is, it is sterile and works in a controlled and sterile atmosphere. The sterility of the user machine 14 can normally be obtained by means of hot sterilization, or low temperature sterilization, for example VHP (Vaporized Hydrogen Peroxide) treatment. In possible further implementations, it is also possible to use sterilization methods that adopt physical barriers in order to ensure the protection of the product, or the containment of the contamination, used in production activities to first of all separate operators from the process such as isolation technology (technology of confined/isolated volumes or environments) as used in machines called isolators. In particular, barriers can be used that are able to guarantee a reciprocal safety from contamination to the operators, from the point of view of exposure to highly toxic products, and to the product, from the point of view of exposure to direct contact with a person/operator, which is the main source of microbiological contamination of sterile products manufactured under aseptic conditions. Unlike traditional aseptic techniques, this technology ensures a high guarantee of sterility of the process obtained through the bio-decontamination of the operating environment with vaporized hydrogen peroxide or other decontaminating agents, such as peracetic acid, ozone or other.

In some embodiments, the method can provide to collect a desired quantity of loose objects to be treated in the feed section 11, which are then transferred to the treatment machine 12. For this purpose, the feed section 11 can comprise a collection container 21 (figs. 2-9) located upstream of the treatment machine 12.

The collection container 21 can be connected to the treatment machine 12 by the feed means 18.

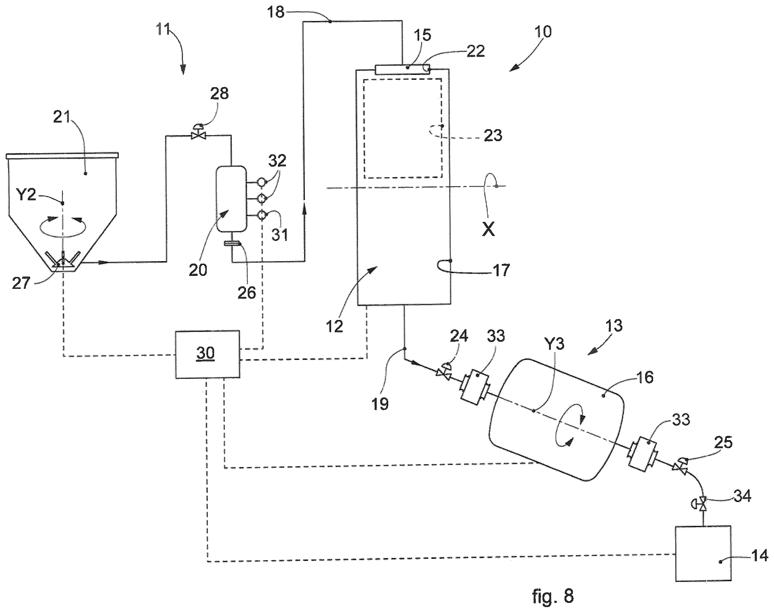

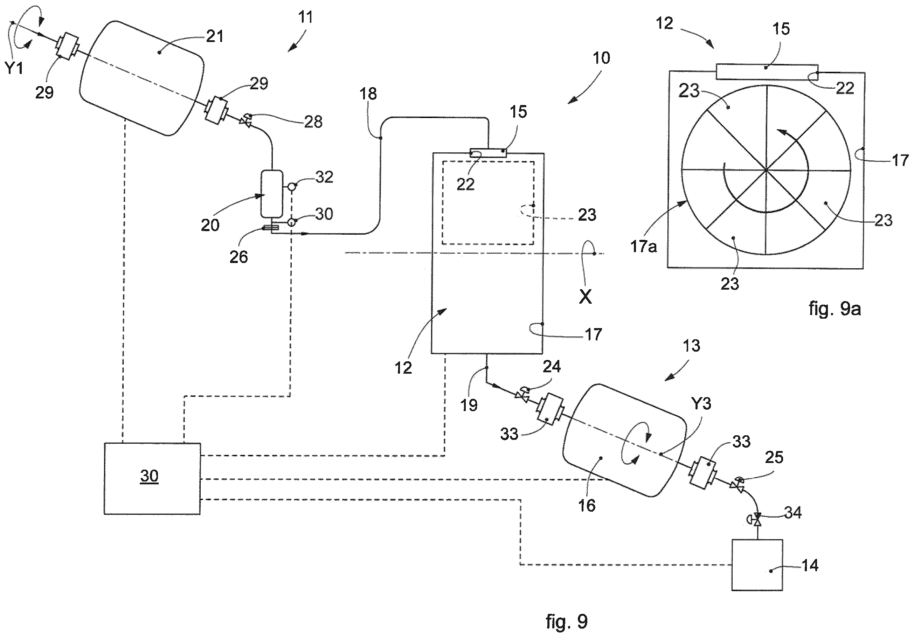

The treatment machine 12 typically provides a treatment chamber 17 inside it (figs. 8, 9, 9a).

The quantity or volume of loose objects collected and to be treated, for example in the collection container 21, can be coordinated with the internal reception or containing capacity of the treatment machine 12, and in particular of the treatment chamber 17.

The method can provide that, during the treatment in the treatment machine 12, the loose objects are kept moving in the treatment chamber 17. For this purpose, the treatment chamber 17 can be static and equipped with shaking means, or it can be mobile, for example rotating. For example, the treatment chamber can be defined by, or include, a rotating drum 17a.

The treatment chamber 17 can be a single chamber, or it can be divided into separate containing compartments or sectors 23. For example, if the treatment chamber 17 is defined by a drum 17a rotating around an axis of rotation X, the rotating drum 17a can have the containing compartments or sectors 23 as above separated and disposed angularly around the axis X.

Therefore, as described by way of example using figs. 8, 9, and 9a, the treatment chamber 17 can provide a single containing compartment or sector 23 or several containing compartments or sectors 23 (fig. 9a) inside which the loose objects to be treated are disposed.

Treatment machines of this type are described for example in international application WO-A-2015150581 in the name of the Applicant.

However, other types of treatment machines 12 with one or more treatment chambers 17 and/or with one or more containing sectors 23 are not excluded.

In some embodiments, the method can provide to feed or directly transfer an entire load or batch of loose objects to be treated, for example introduced or collected in the collection container 21, to the treatment machine 12, inside which they are treated.

In other embodiments, as indicated above, the method can provide a preliminary metering of the loose objects to be treated, that is, to divide a load or batch of loose objects to be treated, for example introduced or collected in the collection container 21, into portions or metered quantities and to sequentially feed, or transfer, the various portions or metered quantities to the treatment machine 12. In these embodiments, the number of portions into which a load or batch of loose objects is divided can advantageously correspond to the number of containing compartments or sectors 23 with which the treatment chamber 17 is provided. Furthermore, the quantity, and therefore the volume, of each of these portions is coordinated with the volumetric capacity of each of the containing compartments or sectors 23.

For example, the method can provide that desired quantities of the loose objects are metered, which are collected as described above in the collection container 21. For example, the metered quantity or volume of loose objects can be a fraction of the quantity of loose objects that is provided or collected in the collection container 21.

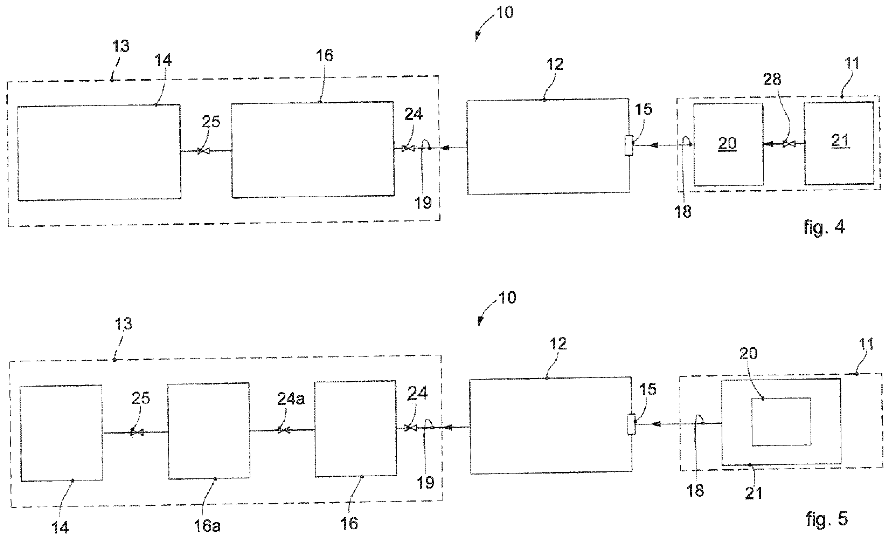

In order to meter portions that have predefined quantities or volumes of the loose objects, before they are transferred to the treatment machine 12 to be treated, the feed section 11 can comprise at least one metering device 20 (figs. 4-9) located upstream of the treatment machine 12.

In possible implementations, the metering device 20 can be outside the collection container 21 and suitably connected thereto. For example, the metering device 20 can be a metering cylinder, of a known volume and predefined to contain an established quantity of loose objects; a quantity which can be advantageously coordinated with the volume or useful capacity of a respective containing sector 23 or of the treatment chamber 17 in general, according to the various embodiments.

In other possible implementations, there can be provided a plurality of metering devices 20, suitably connected on one side to a common collection container 21, or to respective collection containers 21, and on the other side, by means of a suitable predisposition of the feed means 18, to the treatment machine 12, so that they can be used in synchrony to feed the latter. This solution can be advantageous for exploiting the transfer time of a metered quantity from a metering device 20 to the treatment machine 12 in order to, in the meantime, fill the other metering device 20 with the desired metered quantity, thereby efficiently coordinating the metering and transfer times.

For example, in possible implementations the metering device 20 can be between the collection container 21 and the treatment machine 12.

In possible implementations, the metering device 20 can be connected to the treatment machine 12 by means of the feed means 18.

The method, in possible embodiments, can provide to selectively open/close the metering device 20 in order to receive the loose objects, for example from the collection container 21 and/or to transfer the loose objects metered by the metering device 20 to the treatment machine 12.

In possible implementations, in order to selectively transfer the loose objects toward the treatment machine 12, the metering device 20 can, for example, be provided with a release device 26 (figs. 8 and 9), for example provided on the bottom thereof, and configured to open and release the loose objects so that, once the desired metered quantity is reached, they are transferred toward the treatment machine 12 through the feed means 18.

The release device 26 can comprise for example a guillotine valve, or similar interception mean, associated with the bottom of the metering device 20.

The metering device 20 can be equipped with sensors 31, 32 able to detect the presence and/or the level of loose objects inside it. The sensors 31, 32 are advantageously connected to the command and control unit 30. For example, it can be provided to use sensors, such as level and/or presence sensors, of the optoelectronic, electromagnetic, capacitive or other type. To this end, the metering device 20 can for example be provided with at least a transparent part, for example an inspection window, or be made completely transparent, in order to allow the detection by the sensors 31, 32. In particular, a first sensor 31 can be provided which detects the presence/absence of objects inside the metering device 20, thus providing a signal indicating that the metering device 20 is empty or has been emptied. Furthermore, one or more second sensors 32 can be provided, able to provide a signal that a maximum filling level, or an intermediate filling level, has been reached, also according to the type, shape and/or size of the loose objects.

In other possible implementations, in order to selectively receive the loose objects from the collection container 21, the metering device 20, if it is outside the collection container 21, can be associated with an interception element 28 (figs. 4, 6, 7, 8 and 9), for example an on/off valve, connected on one side to the collection container 21 and on the other side to the metering device 20. The interception element 28 is advantageously opened/closed according to requirements for the purposes of transferring the loose objects from the collection container 21 to the metering device 20, in particular being controlled by the command and control unit 30.

Possible embodiments of collection containers 21 and metering devices 20 are described for example in the international application WO 2018/225105 A2 in the name of the Applicant.

In other embodiments, it can be provided that the metering device 20 is integrated inside the collection container 21 (see for example fig. 5). In these embodiments, the metering device 20 can comprise metering elements, such as spoon, shovel, lobe, metering screw elements or similar metering elements disposed inside the collection container 21, in particular on the internal surface thereof. Or, the metering elements can comprise helical guides or portions of helical guides disposed inside the collection container 21, in particular on the internal surface thereof. A combination of these metering elements inside the collection container 21 is also possible. In these embodiments, the relative movement between the collection container 21 and the loose objects present inside it determines, in cooperation with the metering elements, the metering of the desired quantity of loose objects which are sent, by means of the feed means 18, to the treatment machine 12.

As described above, during the treatment the loose objects are temporarily disposed, for the duration of the treatment cycle, in the treatment chamber 17 provided inside the treatment machine 12, where the planned treatment cycle is performed. In order to perform the transfer into the treatment chamber 17, the treatment machine 12 has a loading aperture 22 (figs. 8 and 9) which can be selectively closed with a hermetic seal by the specific closing device 15.

For example, the closing device 15 can be disposed in correspondence or in alignment with the loading aperture 22; for example, the loading aperture 22 and the corresponding closing device 15 can be present on an upper part of the treatment machine 12. The closing device 15 and the loading aperture 22 are therefore associated with the feed means 18.

In possible implementations, the closing device 15 can advantageously comprise a closing and interception mechanism, such as a guillotine or shutter door, and can be associated with specific packings, for example pressurizable packings. In the closed condition the closing device 15 closes with a hermetic seal the loading aperture 22, and therefore the treatment chamber 17, and in the open condition it puts the treatment machine 12 in communication with the feed section 11, allowing the feed of the loose objects to be treated from the feed section 11 to the treatment machine 12 through the loading aperture 22.

According to one embodiment, during the feed, the feed section 11, and therefore the collection container 21 and the metering device 20 in the embodiments in which metering is provided, is selectively put in communication with the treatment machine 12 by activating, that is, opening, the closing device 15 when the interception device 24 downstream of the treatment machine 12 is in the closed condition.

In accordance with the method described here, the feed of untreated loose objects from the feed section 11 to the treatment machine 12 can advantageously be fully automated, controlled by the command and control unit 30.

According to one embodiment, during the feed, the feed section 11 is put in communication with the treatment machine 12 by means of the feed means 18. This can be done selectively, by selective activation of the closing device 15 associated with the loading aperture 22 of the treatment machine 12. The activation comprises opening the loading aperture 22 and enables access to the treatment chamber 17.

According to one embodiment, the treatment of the loose objects and the unloading of the treated loose objects to the unloading section 13 and toward the user machine 14 occurs when the closing device 15 is in the closed condition to close with a hermetic seal the loading aperture 22 and therefore separate the treatment machine 12, and in particular the treatment chamber 17, from the feed section 11, preventing any communication whatsoever, so that there is no contamination of the treated loose objects and of the respective treatment and unloading environments.

According to another embodiment, the feed of the loose objects from the feed section 11 toward the treatment machine 12 occurs, however, when the closing device 15 is in the open condition and the interception device 24 is in the closed condition, thus preventing any communication whatsoever between the unloading section 13 on one side and the treatment machine 12 and feed section 11 on the other, all of which, at this step of the treatment process, can be potentially contaminated, or in any case not controlled and not safe, for example in terms of sterility.

According to one embodiment, at the beginning of a treatment operating cycle it can be provided that, before at least the first feed of the loose objects from the feed section 11 to the treatment machine 12, a pre-treatment of the environment surrounding the loading aperture 22 is performed, so as to limit the contaminations following the insertion of untreated loose objects through the loading aperture 22.

According to further possible embodiments, the transfer of loose objects from the collection container 21, possibly metered by means of the metering device 20, to the treatment machine 12 can occur automatically by drop due to gravity. The feed of loose objects by gravity can be facilitated by tilting the collection container 21 and advantageously by moving the loose objects contained in the collection container 21.

According to another embodiment, which can possibly be combined with the embodiment described above, the transfer and feed of the loose objects to the treatment machine 12 can occur automatically by pneumatic action, for example, by means of a suitable pneumatic device present in, or associated with, the feed section 11. The pneumatic device can be associated with, or be comprised in, the feed means 18 which connect the feed section 11 and the treatment machine 12.

In particular, in some possible implementations the feed by pneumatic action provides to blow air in order to send the loose objects into the treatment chamber 17 by blowing along the feed means 18.

According to further possible implementations, for the purposes of the feed, it can be provided to suck the loose objects to be treated, from the collection container 21 or from the metering device 20 if provided externally, and send them by blowing along the feed means 18 toward the treatment machine 12.

A solution of this type is described for example in the patent application for industrial invention in Italy n. 102019000003773 filed on 14.03.2019 in the name of the Applicant.

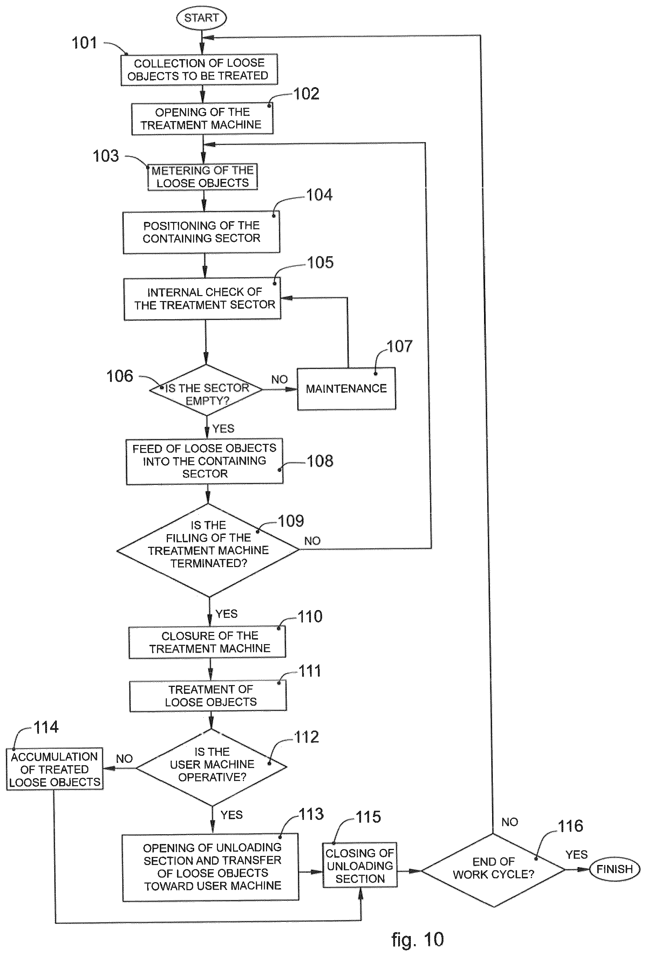

Fig. 10 is used to describe possible embodiments, which can be combined with all the embodiments described here, of the treatment method according to the present description.

According to one embodiment of the method described here and shown by way of example in fig. 10, the feed can provide to collect and accumulate (sub-step 101) the loose objects to be treated before they are sent to the treatment machine 12, for example in the collection container 21 as above.

During the feed, the collection container 21 can be selectively put in communication with the treatment machine 12, possibly after connection with the metering device 20 in the embodiments which contemplate the preliminary metering by means of the metering device 20 outside the collection container 21, for example by activating the interception element 28 to open.

According to one embodiment, the method described here can provide to open the treatment machine 12 (sub-step 102) by opening the closing device 15, in order to allow the transfer of the loose objects to be treated to the treatment machine 12.

Furthermore, according to one embodiment of the method described here, the feed can comprise the intermediate metering (sub-step 103), before the transfer to the treatment machine 12, of a determinate quantity of loose objects coordinated with the reception capacity of the treatment chamber 17 of the treatment machine 12, for example by means of the metering device 20, outside of or integrated with the collection container 21. The metering device 20, if it is outside the collection container 21, can be put in communication with the collection container 21 by opening the interception element 28, at the same time closing the respective release device 26 during the metering.

The metering can occur by means of the metering device 20, outside or inside the collection container 21. In particular, during the metering, in the embodiments in which the metering device 20 is outside the collection container 21, a quantity of loose products is introduced into the metering device 20 which are introduced on each occasion into the treatment chamber 17, whether it is of the type with a single containing sector, or divided into several containing sectors 23, as a function of, for example, the type, shape and/or size of loose objects, the treatment cycle to be carried out or other requirements.

As mentioned, in the case of a treatment chamber 17 divided into several containing sectors 23, the quantity metered on each occasion by the metering device 20, outside or inside the collection container 21, essentially corresponds to the quantity that can be accommodated by one of the containing sectors 23 for the purposes of the treatment.

According to one embodiment, after the metering and during the feed of the loose objects, the metering device 20, when provided outside the collection container 21, is selectively put in communication with the treatment machine 12, for example by activating the release device 26, in order to allow the passage of the metered loose objects toward the treatment machine 12, in particular by means of the feed means 18.

According to one embodiment, the release device 26 is opened when the metering device 20 reaches the correct filling level of loose objects inside it. To this end, the command and control unit 30 can use the signals provided by the one or more second sensors 32.

In these embodiments, the emptying of the metering device 20, at the end of the metering, occurs by opening the release device 26 for a determinate interval of time sufficient for the emptying and for the transfer to the treatment machine 12, in particular by means of the feed means 18. For this purpose, the command and control unit 30 can use the signals provided by the first sensor 31. In the event there is a pneumatic transfer, the opening of the release device 26 is coordinated with the activation of the pneumatic device as above toward the treatment machine 12. The activation of the pneumatic transfer can be provided for a determinate interval of time coordinated with the quantity of loose objects to be transferred, also considering the transit time along the feed means 18.

According to one embodiment, the release device 26 is closed once the quantity of loose objects metered in the metering step is released and transferred to the treatment machine 12.

According to a possible variant, before the metering, the method provides to check that inside the metering device 20, in the embodiments in which it is outside the collection container 21, there are no residual loose objects, advantageously using the first sensor 31, which supplies a correlated signal to the command and control unit 30.

According to a further embodiment, the metering function is subjected to a check to verify that the quantity of metered loose objects reaches a predefined level or value. To this end, the one or more second sensors 32 can be used, which send a corresponding signal to the command and control unit 30. In this way, during the metering there is a check to verify the correct filling level is reached inside the metering device 20, also on the basis of the type, shape and/or size of loose objects to be treated and possibly the reception capacity of the treatment chamber 17 or of the containing sectors 23, where provided.

According to another variant, it can be provided that the transfer of the metered loose objects from the metering device 20 to the treatment machine 12 is subject to a check to verify that the metering device 20 has been completely emptied of the loose objects present therein, that is, that the transfer of the metered loose objects to the treatment machine 12 has been completed. This check can be carried out by means of the first sensor 31. Consequently, when the first sensor 31 supplies a signal to the command and control unit 30 signaling that the emptying of the metering device 20 has occurred, the transfer function, for example in this specific case the pneumatic transfer function, can be stopped in a coordinated manner. The stopping time can advantageously be coordinated with the transit and emptying time also of the feed means 18, so that adequate time is available for the loose objects to be introduced into the treatment machine 12.

Advantageously, the method provides that the check on the level of loose objects metered in the metering device 20, by means of the one or more second sensors 32, and the check that the emptying has occurred, by means of the first sensor 31, are managed by the command and control unit 30 and that the activation of the interception element 28 and of the release device 26, respectively to open and to close, is also selective as a function of this information processed by the command and control unit 30.

According to possible embodiments, the method provides that, before and/or during the feed of the loose objects toward the treatment machine 12, the latter are kept moving, or shaking, inside the collection container 21. This effect can be obtained, according to possible variants, by moving, in particular by rotating, the same collection container 21 (fig. 9), for example by rotating it on itself along its own longitudinal axis Y1, or by keeping the collection container 21 static and using shaking means 27 present inside it, rotating around an axis Y2 (fig. 8).

Advantageously, keeping the loose objects moving or shaking in the collection container 21 facilitates their transfer toward the treatment machine 12, or toward the metering device 20 in the embodiments in which the metering is provided by means of a metering device 20 outside the collection container 21, since it prevents the loose objects from cramming together, compacting and massing together in the same collection container 21, which would block their exit from the latter.

According to a possible implementation, the movement of the loose objects inside the collection container 21 by rotation of the collection container 21 can be facilitated by the presence, inside the collection container 21, of one or more shaped guides, for example with a helical shape or with helical-shaped portions, or spoon, shovel, lobe, metering screw or similar elements, associated with the internal walls of the collection container 21. In this case, these elements can act as metering elements, as indicated above in some embodiments.

In the embodiments in which the loose objects are moved by rotating the collection container 21, it can be connected to the feed means 18 by means of at least one rotating joint 29 (fig. 9) which allows the relative rotation of the collection container 21 with respect to the feed means 18.

In some embodiments, it can be provided to adjust the speed of rotation of the collection container 21 in a manner coordinated with how much the collection container 21 is filled, also in consideration of its progressive emptying. Indicatively, as the filling of the collection container 21 decreases, as the loose objects are removed, the speed of rotation of the collection container 21 itself can increase. This can allow a desired uniformity in the transfer flow rate of the desired quantities of loose objects toward the treatment machine 12, and in particular the metering device 20 where provided according to the embodiments described here.

Advantageously, the rotation of the collection container 21 also facilitates the exit and transfer of the loose objects toward the metering device 20, where provided, and toward the feed means 18, this in particular if gravity is used to determine the passage of the loose objects from the collection container 21 to the possible metering device 20 and to the feed means 18, as described above. Consequently, the number of rotation revolutions of the collection container 21 is correlated to the quantity of loose objects that are transferred.

In the embodiments in which the movement of the loose objects inside the collection container 21 occurs by means of shaking means 27 (fig. 8), the latter are means suitable to shake and/or mix the loose objects inside the collection container 21 and can for example be blades, anchors, screws or portions of screws, arms or other suitable shaking components.

In the embodiments in which the metering is carried out by means of the metering device 20 outside the collection container 21, the metering occurs, therefore, having opened the interception element 28, while the release device 26 is closed and the loose objects are moved, or shaken, in the collection container 21, facilitating their transfer.

According to one embodiment, the feed of the loose objects from the collection container 21 to the treatment machine 12 occurs, possibly after metering in the embodiments in which it is contemplated, when the closing device 15 is opened, thereby enabling the introduction of the loose objects to be treated into the treatment machine 12. These operations can also be coordinated with the movement, or shaking, of the loose objects in the collection container 21.

Furthermore, in the embodiments that provide the metering with an external metering device 20, the feed of the loose objects from the metering device 20 to the treatment machine 12 occurs when the interception element 28 is closed, the release device 26 is opened and the closing device 15 is in the open condition, enabling the introduction into the treatment machine 12. In the embodiments in which the collection container 21 is moved to facilitate the advancement of the loose objects, the closure of the interception element 28 in this context is advantageously coordinated with stopping the movement of the collection container 21, in order to prevent the objects from continuing to be conveyed toward the metering device 20 while the interception element 28 is closed, which could subsequently hinder the correct outflow of the loose objects.

According to further possible embodiments which include the metering, the method can provide that the metered loose objects are kept moving, or shaking, also inside the metering device 20. This can be advantageous in order to prevent also in this case unwanted cramming and compacting of the loose objects in the metering device 20, which would prevent their correct transfer to the treatment machine 12. This movement can be obtained by moving the metering device 20, which for example can be made to rotate around its own axis, or by keeping the metering device 20 static and providing shaking means present inside the metering device 20.

In the embodiments in which the treatment chamber 17 is equipped with, or has, a plurality of containing sectors 23 (for example fig. 9a), the feed of the loose objects to the treatment machine 12 is coordinated with the rotation of the drum 17a and the positioning, on each occasion, of a containing sector 23 in a position to receive the loose objects in correspondence, or in alignment, with the loading aperture 22 (sub-step 104).

According to one embodiment, the positioning of the containing sector 23 in the correct position to receive the loose objects can occur before, or during, the metering of the loose objects, so that the containing sector 23 is in a waiting condition aligned with the loading aperture 22 and ready to receive the metered loose objects.

Each containing sector 23 is typically equipped with a respective closing door, which can be selectively opened/closed as a function of the reception of the loose objects; in particular, it opens when the containing sector 23 is ready to receive the loose objects and closes when the complete transfer of the desired metered quantity of loose objects has occurred. Advantageously, the progressive positioning of the containing sectors 23 with respect to the loading aperture 22, as well as their selective opening/closing, are managed automatically by means of the command and control unit 30.

According to another embodiment, the method described here can comprise, before the feed of the loose objects from the feed section 11 to the treatment machine 12, a preliminary check or inspection of the treatment machine 12, and in particular of the containing sectors 23 where provided, in order to verify the presence/absence of at least any residual loose objects arriving from a previous treatment cycle in the treatment machine 12.

In particular, before enabling the transfer of the metered loose objects to the treatment chamber 17, or to the respective containing sector 23 positioned in correspondence with the loading aperture 22, the method provides to check or inspect the inside of the treatment chamber 17, or of the corresponding containing sector 23 (sub-step 105). The preliminary check or inspection comprises, in particular, verifying the presence/absence of loose objects of the previous treatment inside the containing sector 23 to be filled, in order to ascertain the absence of loose objects from the previous treatment, before proceeding with the feed.

Advantageously, this preliminary check allows to prevent contaminations and mixing between residual loose objects, that is, ones which have remained undesirably present, for various reasons, inside the treatment chamber 17 from the previous treatment, and the loose objects to be treated in the subsequent treatment. These mixtures of non-homogeneous loose objects can lead to malfunctions and even stop of the user machine 14 with consequent, and possibly considerable, economic and productivity losses.

The preliminary check or inspection can be carried out by means of a control or inspection assembly, for example comprising one or more detection or inspection devices, for example, optical devices, digital cameras or similar or comparable devices, which cooperate with the command and control unit 30.

With reference to embodiments of the method described using fig. 10, a check is therefore provided (sub-step 106) of the outcome of the preliminary check of sub-step 105. If the containing sector 23 is not empty, an intervention is carried out, for example, to remove the objects present inside the containing sector 23 (sub-step 107). In particular, it can be provided to send an alarm signal to the command and control unit 30 and to implement measures suitable to remove the unwanted loose objects, for example by means of a cleaning fluid, such as compressed air, which forces the unwanted loose objects toward a specific exit of the treatment machine 12, or opening a maintenance door by means of which to remove the unwanted loose objects, or other operations suited to the purpose.

If, on the other hand, the verification of the preliminary check detects the absence of loose objects from the previous treatment in the containing sector 23, the feed of the loose objects (sub-step 108) is performed, possibly after metering in the embodiments which provide it, from the feed section 11 to the treatment machine 12, in particular to the containing sector put on each occasion in cooperation with the loading aperture 22.

According to one embodiment, the containing sector 23, once filled, is closed and the drum 17a is rotated so that a new containing sector 23 faces toward the loading aperture 22. At this point, if the treatment chamber 17 is of the type with a plurality of containing sectors 23, the steps described above for each containing sector 23 are repeated, until the whole batch of loose objects to be treated, for example originally contained in the collection container 21, has been loaded into the treatment machine 12.

In particular, during the feed, the method provides to make the containing sectors 23 rotate progressively and stepwise, for example by means of corresponding rotation of the rotating drum 17a described above, in order to put on each occasion a containing sector 23 in the correct position to receive a determinate quantity of loose objects, advantageously in coordination with progressive metering operations which are carried out by means of the metering device 20.

With each rotation of the drum 17a and for each empty containing sector 23, the preliminary check (sub-step 105) is then performed before feeding the loose objects inside each containing sector 23 (sub-step 108).

A check is then carried out to verify whether the treatment machine 12 has been completely fed (sub-step 109).

In particular, the command and control unit 30 is programmed to repeat the loading of the loose objects into the various containing sectors 23, repeating the sub-steps from 103 to 109, until the whole batch of loose objects to be treated, present in the collection container 21 and metered on each occasion by means of the metering device 20, is transferred into the containing sectors 23, thus completing the feeding step.

Once the feeding step is finished, the treatment chamber 17 and, in particular, the respective containing sectors 23 where provided, have been filled, the check of the sub-step 109 gives a positive result and the sealing of the loading aperture 22 (sub-step 110) is then provided by means of the closing device 15: at this point the treatment machine 12 is closed and is no longer in communication with the feed section 11.

The treatment cycle (step 111) of the loose objects is then started and completed. During the execution of the treatment cycle, the treatment machine 12 is normally kept closed with a hermetic seal and separated both with respect to the feed section 11, by means of the closing device 15, and also with respect to the unloading section 13, by closing with the interception device 24.

According to a possible embodiment, however, the method can provide that, during a treatment cycle, the treatment machine 12 is indeed kept closed and separated with respect to the feed section 11, by means of the closing device 15, but not with respect to the unloading section 13, keeping the interception device 24 open. In this way, it is possible to also subject the environments of the unloading section 13 to treatment. This operating mode can be implemented as needed, or cyclically, within the scope of a defined work program, in order to treat the unloading section 13, based on requirements.

At the end of a treatment cycle, the method provides to automatically unload the loose objects from the treatment machine 12 into the unloading section 13.

The unloading step can provide, for example, that the containing sectors 23 are progressively positioned in cooperation with the unloading means 19.

According to the present invention, the unloading step occurs by opening the interception device 24 and keeping the closing device 15 closed with a hermetic seal so that the treatment machine 12 on one side is in communication with the unloading section 13 and on the other side is not in communication with the feed section 11. In this way, the controlled conditions of the environments downstream of the treatment machine 12, and in particular of the unloading section 13 and advantageously of the user machine 14 operatively associated therewith, are preserved.

In accordance with some embodiments, the method provides to manage the unloading of the treated loose objects, at the end of the treatment cycle, on the basis of a verification (sub-step 112) of whether the downstream user machine 14 is operational and therefore needs, or doesn't need, to be fed with the treated loose objects.

In particular, before the unloading, the method provides to check the state of operation of the user machine 14, the unloading of the treated loose objects being conditioned on the basis of the outcome of the check on the state of operation.

By "operating user machine 14" or "user machine 14 in a state of operation" we mean that the user machine 14 is active and in production and is not in a state of machine downtime, that the objects treated and transferred to the user machine 14 are correct at the end of the work cycle thereof and that the speed at which the treated loose objects are unloaded from the treatment machine 12 to the user machine 14 meets the productivity criteria of the latter.

In particular, the feed of the loose objects to be treated from the feed section 11 to the treatment machine 12, the treatment of the loose objects in the treatment machine 12 and the unloading of the loose objects from the treatment machine 12 to the unloading section 13 are carried out on the basis of the state of operation of the user machine 14. Advantageously, the command and control unit 30 is in communication with the user machine 14, receiving from the latter information relating to its operation, so as to manage and control the treatment method described here in an automated and coordinated manner.

According to one embodiment, if the user machine 14 is operative and requires a feed of treated loose objects, the check of sub-step 112 is positive and the unloading is provided toward the unloading section 13 and from there to the user machine 14 (sub- step 112).

According to one embodiment, the method described here provides that the unloading always occurs with the closing device 15 in a closed condition so as not to contaminate the unloading section 13.

According to possible implementations, the method described here can also provide an operation of blowing a fluid, for example compressed air, preferably treated and controlled air, in particular sterile air, to facilitate the exit and the unloading of the loose objects from the treatment machine 12 toward the unloading section 13.

In one variant, which for example may not contemplate the presence of the accumulation container 16, the treated loose objects can be transferred directly from the unloading section 13 to the user machine 14 (for example fig. 2).

In other variants, the treated loose objects can be transferred and accumulated in the accumulation container 16 (for example figs. 3-9) and then be transferred, according to operating requirements, to the user machine 14 (sub-step 113).

In the event the check of sub-step 111 is not positive, the treated loose objects can in any case be transferred to the unloading section 13 where they can for example be kept accumulated in the accumulation container 16 (sub-step 114).

In some embodiments, the method can therefore provide that the accumulation of the loose objects treated by the treatment machine 12 in the unloading section 13 occurs by suitably driving the interception device 24, which selectively does or doesn't enable the passage of the loose objects through the unloading means 19. The selective drive is advantageously managed by the command and control unit 30. The opening of the interception device 24 in order to put the treatment machine 12 in communication with the unloading section 13 is also coordinated by the command and control unit 30 with the maintenance of the sealed condition of the closing device 15.

The method can also provide that, during the accumulation of treated loose objects in the accumulation container 16 of the unloading section 13, there is no communication between the accumulation container 16 and the user machine 14 located downstream. To this end, another interception device 25 (figs. 3-9) can be provided downstream of the accumulation container 16 to selectively intercept or release the treated loose objects toward the user machine 14.

The embodiments that provide accumulating the treated loose objects in the accumulation container 16 are particularly advantageous in the event the user machine 14 is a filling machine used for example in the pharmaceutical industry, or in any case a machine that works continuously without interruptions, of the type used for example in the field of pharmaceutical industry. In this way, it is possible, in fact, to suitably coordinate the production, therefore the treatment, capacity of the treatment chamber 17 with the productivity of the user machine 14.

According to one embodiment, the method can provide moving, or shaking, the treated loose objects present in the accumulation container 16 so as to allow a uniform passage and transfer of loose products from the accumulation container 16 to the user machine 14, preventing the loose objects from adhering to each other, cramming together and compacting, and therefore preventing the exit from the accumulation container 16 from becoming blocked. The movement or shaking can occur by moving the accumulation container 16, which for example can be rotated around its own longitudinal axis Y3, or by keeping the accumulation container 16 static and using suitable shaking means (not shown) present in the accumulation container 16 itself.

According to one embodiment, the movement of the loose objects inside the accumulation container 16 can also be advantageously used for the purpose of facilitating and assisting the unloading of the loose objects from the accumulation container 16 to the user machine 14.

In the embodiments in which the movement of the loose objects inside the accumulation container 16 occurs by rotating the accumulation container 16, the latter can have one or more guides inside it, for example with a helical shape or with helical-shaped portions, integral with the internal walls of the accumulation container 16 itself or spoon, shovel, lobe, metering screw elements or suchlike, associated with the internal walls of the accumulation container 16. In the event that, as mentioned above, the method also provides to meter the loose objects treated by the treatment machine 12 so that they can be transferred in a metered manner to the user machine 14, these elements can act as metering elements, as indicated above in some embodiments. In these embodiments, a relative movement between the accumulation container 16 and the treated loose objects present inside it determines, in cooperation with the metering elements, the metering of the desired metered quantity of loose objects which is sent on each occasion to the user machine. 14.

Furthermore, if the movement of the loose objects occurs by rotating the accumulation container 16, the accumulation container 16 can be connected to the unloading means 19 by means of at least one rotating joint 33 (figs. 8, 9) which allows the relative rotation of the accumulation container 16 with respect to the unloading means 19.

According to some embodiments, the number of rotation revolutions of the accumulation container 16 is correlated to the quantity of loose objects which are progressively unloaded and transferred toward the user machine 14, so as to advantageously guarantee a continuous feed to the latter, in order to satisfy its operational requirements.

According to one embodiment, the unloading of the treated loose objects from the accumulation container 16 to the user machine 14 occurs when the interception device 25, provided between the accumulation container 16 and the user machine 14, enables the passage along the unloading means 19, the interception device 24 is in the closed condition and the loose objects are advantageously moved inside the accumulation container 16 to facilitate their transfer toward the user machine 14.

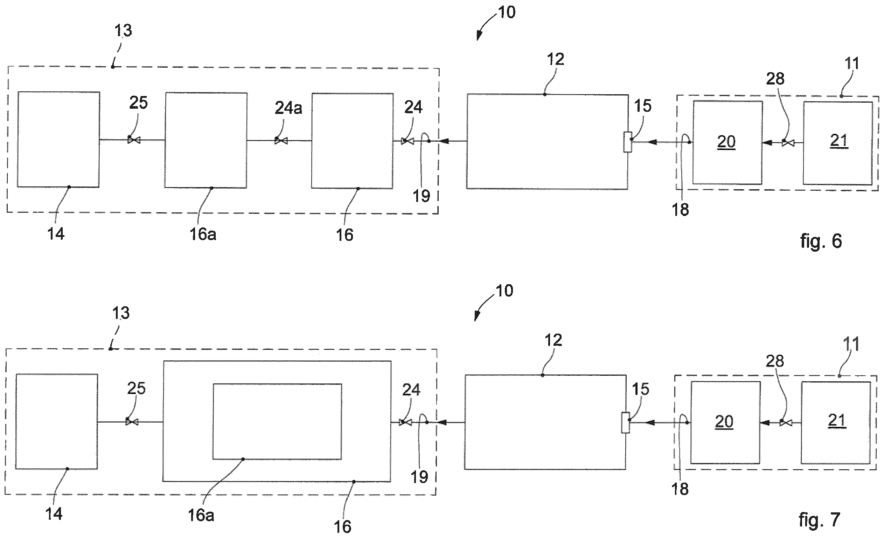

In some embodiments, described using figs. 5 and 7, the method can provide, as already indicated above, a predefined metering of the treated loose objects before they are transferred to the user machine 14. For this purpose, the unloading section 13 can include a respective metering device 16a. The metering device 16a can be outside and downstream of the accumulation container 16, before the user machine 14 and the corresponding interception device 25 (fig. 5). In this case, a valve device 24a can be provided between the accumulation container 16 and the metering device 16a, whose functioning and operation can be similar to that of the interception element 28. Alternatively, the metering device 16a can be integrated inside the accumulation container 16 (fig. 7), made for example as metering elements described above. The embodiments of the metering device 16a, whether it is respectively outside the accumulation container 16 or integrated with it, can be made like the respective embodiments of the metering device 20 as above, which can also be outside or integrated with the respective collection container 21.

According to one embodiment, during the unloading, the distribution of the loose objects to the user machine 14 can also be provided by means of a vibrating container (not shown) which allows the homogeneous and uniform separation of the loose objects before the unloading into the user machine 14.

The vibrating container can be associated with a sensor that detects when the user machine 14 can receive loose objects, thus allowing their selective release toward the user machine 14.

According to one embodiment, during the unloading, the controlled and homogeneous distribution of the loose objects to the user machine 14 can be provided by means of a selector device 34 located upstream of the user machine 14. The selector device 34 can be a motorized valve provided, for example, with one or more paddles able to slow down the flow of loose objects transferred, gradually distributing and feeding the same loose objects to the user machine 14.

According to one embodiment, the step of controlled distribution through the separator device 34 can occur before the distribution step in the vibrating container.

According to one embodiment, once the unloading of the loose objects from the treatment machine 12 to the unloading section 13 and from there possibly to the user machine 14 has terminated, the interception device 24 is closed (sub-step 115). At this point, the method can be resumed from sub-step 102.

In particular, according to one embodiment, once the unloading of the loose objects from the treatment machine 12 to the unloading section 13 has terminated, the interception device 24 is closed (sub-step 114) and a check is carried out by the command and control unit 30 on whether the work cycle has been terminated (sub-step 116). If so, the method ends, otherwise it starts again from sub-step 102 of opening the treatment machine 12 by activating the closing device 15, in order to resume feeding the loose objects to be treated from the feed section 11 to the treatment machine 12.

Some embodiments can provide executing various steps, passages and operations, as described above. These steps, passages and operations can be carried out with instructions executed by a machine or an apparatus that cause the execution of certain steps by a general-purpose or special-purpose processor. Alternatively, these steps, passages and operations can be performed by specific hardware components which contain hardware logic to carry out the steps, or by any combination whatsoever of programmed computer components and customized hardware components.

The command and control unit, or system controller, 30 can typically comprise a central processing unit, or CPU, an electronic memory, an electronic database and auxiliary circuits (or I/O) (not shown). For example, the CPU can be any form of computer processor that can be used in the IT field for process control. The memory can be connected to the CPU and can be one or more of those commercially available, such as a random access memory (RAM), a read only memory (ROM), a floppy disk, a hard disk, a mass memory, or any other form of digital archiving, local or remote. The software instructions and data can for example be coded and stored in memory in order to command the CPU. The auxiliary circuits can also be connected to the CPU to help the processor in a conventional manner. The auxiliary circuits can include for example at least one of either: cache circuits, feed circuits, clock circuits, input/output circuitry, subsystems, and suchlike. A program (or computer instructions) readable by the command and control unit, or system controller, 30 can determine which tasks are achievable in accordance with the method according to the present description.

Embodiments of the method in accordance with the present description can also be included in a computer program storable in a non-transient data support readable by a computer which contains the instructions which, once executed by a treatment apparatus 10 which includes a treatment machine 12, a feed section 11 and an unloading section 13, advantageously controlled and managed by the command and control unit, or system controller, 30 determine the execution of the method in question.

Furthermore, elements according to the present invention can be provided as means readable by a machine or apparatus for storing the instructions executable by the machine or apparatus. The readable means can include, but are not limited to, floppy disks, optical disks, CD-ROMs, and magneto-optical disks, ROMs, RAMs, EPROMs, EEPROMs, optical or magnetic cards, solid state mass storage, propagation means or other types of means readable by a machine or apparatus suitable to store electronic information. For example, the present invention can be downloaded as a computer program that can be transferred from a remote computer (for example a server) to a computer that makes a request (for example client), by means of data signals made with wave carriers or other propagation means, via a communication link (for example a modem or a network connection).

It is clear that modifications and/or additions of parts or steps may be made to the method to treat loose objects as described heretofore, without departing from the field and scope of the present invention. It is also clear that, although the present invention has been described with reference to some specific examples, a person of skill in the art shall certainly be able to achieve many other equivalent forms of treatment of loose objects, having the characteristics as set forth in the claims and hence all coming within the field of protection defined thereby.

In the following claims, the sole purpose of the references in brackets is to facilitate reading: they must not be considered as restrictive factors with regard to the field of protection claimed in the specific claims.

## Claims

1. Method to treat loose objects, wherein the treatment includes at least the sterilizing of said loose objects, said method comprising:
- feeding untreated loose objects from a feed section (11) to a treatment machine (12);
- treating the untreated loose objects inside the treatment machine (12);
- unloading the treated loose objects from the treatment machine (12) into an unloading section (13) associated with a machine (14) that uses the treated loose objects, wherein said machine (14) is kept in controlled operating conditions, that is, it is sterile and works in a controlled and sterile atmosphere, obtained by means of hot sterilization, or low temperature sterilization, such as VHP (Vaporized Hydrogen Peroxide) treatment, and/or by using sterilization methods that adopt physical barriers;
wherein said method further provides that:
- before the treatment, when the treatment machine (12) is in a condition in which it contains untreated loose objects, said treatment machine (12) is put in communication, for the purpose of feeding, with the feed section (11) and not with the unloading section (13); and that
- at the end of the treatment, when the treatment machine (12) is in a condition in which it contains treated loose objects, said treatment machine (12) is put in communication, for the purpose of unloading, with the unloading section (13) and not with the feed section (11),
wherein the execution of the treatment method is managed and controlled automatically by means of a command and control unit (30) and
wherein during the treatment of said objects, the treatment machine (12) is separated, and therefore is not put in communication, at least with respect to said feed section (11) and also with respect to said unloading section (13), such that, during the whole treatment, the unloading section (13) is never in communication, direct or indirect, with the feed section (11),
wherein the method provides to manage the unloading of the treated loose objects, at the end of the treatment cycle, on the basis of a verification of whether the downstream user machine (14) is operational and therefore needs, or does not need, to be fed with the treated loose objects by the unloading section (13), wherein, if the verification is positive, the method provides to open the unloading section and transfer the loose objects toward the machine (14), and
wherein, if the verification is not positive, the method provides, during unloading, to accumulate, in the unloading section (13), the treated loose objects unloaded from the treatment machine (12).

2. Method as in claim 1, **characterized in that** during feeding, the feed section (11) is selectively put in communication with the treatment machine (12) by selective opening of a closing device (15) associated with a loading aperture (22) of the treatment machine (12).

3. Method as in claim 2, **characterized in that** the treatment of the loose objects and the unloading of the treated loose objects to the unloading section (13) and toward the user machine (14) takes place when said closing device (15) is in a closed condition, sealing the loading aperture (22).

4. Method as in claim 2 or 3, **characterized in that**, during unloading, the unloading section (13) is selectively put in communication with the treatment machine (12) by selectively opening an interception device (24) associated with unloading means (19) which connect the treatment machine (12) with the unloading section (13), with the provision that the closing device (15) is in the closed condition.

5. Method as in claim 4, **characterized in that**, during feeding, the feed section (11) is selectively put in communication with the treatment machine (12) by opening the closing device (15) when the interception device (24) is in a closed condition.

6. Method as in any claim hereinbefore, **characterized in that** the feed provides the intermediate metering, before the transfer to said treatment machine (12), of determinate quantities of loose objects coordinated with an internal reception capacity of the treatment machine (12).

7. Method as in any claim hereinbefore, **characterized in that**, before feeding the loose objects from the feed section (11) to the treatment machine (12), a preliminary inspection check of the inside of said treatment machine (12) is provided, to verify the presence/absence at least of possible residual loose objects arriving from a previous treatment cycle in said treatment machine (12).

8. Method as in any claim hereinbefore, **characterized in that**, before unloading, it provides to check the state of operation of the user machine (14), said unloading of the treated loose objects being conditioned according to the result of said check on the state of operation.

9. Method as in any claim hereinbefore, **characterized in that** it provides, during unloading, to accumulate, in the unloading section (13), the treated loose objects unloaded from the treatment machine (12), before they are transferred to the user machine (14).

10. Method as in any claim hereinbefore, **characterized in that** it provides, during unloading, the metering of determinate quantities of the treated loose objects unloaded from the treatment machine (12) before they are transferred to the user machine (14).

11. Computer program which can be memorized in a non-transient data support that can be read by a computer that contains the instructions which, once performed by a treatment apparatus (10) which includes a treatment machine (12), a feed section (11) and an unloading section (13), determine the execution of the method as in any claim from 1 to 10.
